(19) Europäisches Patentamt
European Patent Office
Office européen des brevets

(11) **EP 3 051 271 A1**

(12) **EUROPÄISCHE PATENTANMELDUNG**

(43) Veröffentlichungstag:
**03.08.2016 Patentblatt 2016/31**

(51) Int Cl.:
**G01N 21/27** (2006.01)   **G01N 21/31** (2006.01)
**G01N 33/49** (2006.01)

(21) Anmeldenummer: **15152606.8**

(22) Anmeldetag: **27.01.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **Siemens Healthcare Diagnostics Products GmbH**
**35041 Marburg (DE)**

(72) Erfinder: **Sass, Karl**
**35274 Kirchhain (DE)**

(54) **Verfahren zur Bestimmung von Lipiden und anderen Störsubstanzen in Körperflüssigkeitsproben**

(57) Die vorliegende Erfindung betrifft ein Verfahren und ein automatisches Analysegerät zum genaueren Bestimmen der Konzentration von Lipiden und anderen Störsubstanzen in Körperflüssigkeiten, insbesondere von Störsubstanzen wie Bilirubin und Hämoglobin in Blutserum- und Blutplasmaproben.

FIG 1

EP 3 051 271 A1

**Beschreibung**

Die vorliegende Erfindung betrifft ein Verfahren und ein automatisches Analysegerät zum Bestimmen der Konzentration von Lipiden und anderen Störsubstanzen in Körperflüssigkeiten, insbesondere von Störsubstanzen wie Bilirubin und Hämoglobin in Blutserum- und Blutplasmaproben.

Zahlreiche Nachweis- und Analyseverfahren zur Bestimmung physiologischer Parameter in Körperflüssigkeitsproben beruhen auf photometrischen Messprinzipien. Photometrische Verfahren ermöglichen den qualitativen und quantitativen Nachweis von Analyten in flüssigen Proben.

Die Bestimmung klinisch relevanter Parameter, wie zum Beispiel der Konzentration oder der Aktivität eines Analyten erfolgt vielfach, indem ein Aliquot einer Körperflüssigkeit eines Patienten mit einem oder mehreren Testreagenzien in vitro vermischt wird, wodurch eine biochemische Reaktion in Gang gesetzt wird, die eine messbare Veränderung einer optischen Eigenschaft des Testansatzes bewirkt. Die Photometrie untersucht und nutzt die Schwächung eines Lichtstroms beim Durchtritt durch ein absorbierendes und/oder streuendes Medium. Je nach Art der ausgelösten biochemischen oder biophysikalischen Reaktion kommen unterschiedliche photometrische Messverfahren zum Einsatz, die die Messung eines trüben flüssigen Testansatzes ermöglichen.

Hierzu können turbidimetrische Verfahren eingesetzt werden, bei denen die Trübung beziehungsweise die optische Dichte einer Lösung oder Suspension anhand der Lichtschwächung oder Extinktion eines direkt durch die Suspension hindurch tretenden Lichtstrahls gemessen wird.

Die Intensität des Lichtstrahls nimmt beim Durchtritt durch eine Messzelle beziehungsweise Küvette, die eine flüssige Probe enthält, ab. Die Verluste können durch Interaktionen des Lichtstrahls mit der in der Messzelle befindlichen Probe, beispielsweise durch Absorptions-, Diffraktions-, Streuungs- und/oder Reflexionseffekte beeinflusst werden. Im Allgemeinen können Diffraktions-, Beugungs- und Reflexionseffekte vernachlässigt beziehungsweise durch Referenzmessungen ausgeglichen werden, so dass hauptsächlich die Absorption zur Schwächung des Lichtstrahls beiträgt.

Photometrische Konzentrationsbestimmungen beruhen daher auf einer gesetzmäßigen Abhängigkeit der Extinktion beziehungsweise Absorption von der Konzentration der gelösten Stoffe und der Schichtdicke der Messzelle bei einer bestimmten Wellenlänge des eingestrahlten Lichts. Diesen Zusammenhang beschreibt das Lambert-Beersche Gesetz:

$$E(\lambda) = -\log(I/I_0) = \varepsilon(\lambda) \cdot c \cdot d$$

wobei $E(\lambda)$ die von der Wellenlänge $\lambda$ des Lichtstrahls abhängige Extinktion, $I$ die Lichtintensität nach Durchtritt durch die Probe, $I_0$ die Lichtintensität vor Durchtritt durch die Probe, $\varepsilon(\lambda)$ der wellenlängenabhängige molare Extinktionskoeffizient eines durchstrahlten Stoffes, $c$ die molare Konzentration des durchstrahlten Stoffes und $d$ die durch den Lichtstrahl durchstrahlte Schichtdicke, beispielsweise der Messzelle ist.

Anhand der Extinktion $E(\lambda)$ einer Probe lässt sich die Konzentration einer Substanz in einer Lösung ermitteln. Dazu ist es erforderlich, dass zuvor die Extinktion mindestens einer Standardlösung bekannter Konzentration bestimmt wurde. Da sich die Extinktion proportional zur Konzentration verhält, kann mittels Kalibration durch Extinktionsmessungen mehrerer Standardlösungen bekannter Konzentrationen die Konzentration einer gelösten Substanz in einer unbekannten Probe ermittelt werden.

Die Extinktion einer Probe hängt jedoch nicht nur von der Konzentration der zu bestimmenden Substanz selbst ab, sondern auch von der Art der Probenmatrix. Die Extinktionen verschiedener Substanzen verhalten sich in einem Gemisch additiv, sofern die Substanzen nicht untereinander wechselwirken. Körperflüssigkeiten, wie beispielsweise Blutplasma oder Blutserum sind jeweils komplexe Gemische und enthalten neben dem zu bestimmenden Analyten eine Vielzahl weiterer Substanzen, die die Gesamtabsorption der Probe beeinflussen.

Körperflüssigkeitsproben können jedoch in Einzelfällen abnormal hohe Konzentrationen einer oder mehrerer intrinsischer, also körpereigener Substanzen enthalten, die sich bei Überschreitung einer tolerablen Konzentration in photometrischen Detektionsverfahren als störend erweisen und sich zu einem systematischen Fehler auswirken können.

Probleme bereiten bekanntermaßen hämolytische, ikterische und/oder lipämische Serum- oder Plasmaproben, die über abnormal hohe Hämoglobin-, Bilirubin- und/oder Lipid-Konzentrationen verfügen. Abnormal hohe Konzentrationen dieser interferierenden Substanzen können durch einen pathologischen Zustand des Patienten oder aber durch eine unsachgemäße Probengewinnung oder -lagerung verursacht werden. Werden solche Proben einem photometrischen Verfahren unterworfen, das der Bestimmung eines analytischen, diagnostisch relevanten Parameters dient, besteht die Gefahr einer Fehlbestimmung die gegebenenfalls eine Fehldiagnose und schlimmstenfalls eine Fehlbehandlung des Patienten zur Folge haben kann. Die präanalytische Identifikation hämolytischer, ikterischer sowie lipämischer Proben ist also zur Vermeidung von fehlerhaften Analyseergebnissen von besonderer Wichtigkeit.

Es besteht daher ein Bedarf an Verfahren zur Ermittlung der spektrometrischen Auswirkungen störender Substanzen in Körperflüssigkeitsproben bzw. zur Identifizierung von Körperflüssigkeitsproben, die erhöhte Konzentrationen einer oder mehrerer Störsubstanzen enthalten.

In EP-A1-1059522, US 4,263,512, US 2009/0009750 A1 und US 2010/0174491 A1 sind verschiedene Verfahren zur Bestimmung von Bilirubin, Hämoglobin und Lipiden in Plasma- oder Serumproben beschrieben. In der EP-A1-1059522 wird beispielsweise die nach Abzug der Extinktion durch Hämoglobin und Bilirubin verbleibende Extinktion, die insbesondere auch die durch Lipide verursachte Extinktion enthält, lokal linear approximiert.

Auch das letztgenannte Verfahren hat jedoch den Nachteil, dass gerade eine vergleichsweise hohe Lipidkonzentration die Bestimmung von Bilirubin und Hämoglobin in derselben Probe beeinflussen und somit die Messwerte verfälschen kann.

In der WO 2013/010970 A1 ist bereits ein Verfahren beschrieben, dass auch in Gegenwart hoher Lipidkonzentrationen eine genaue Bestimmung von Bilirubin und Hämoglobin und auch eine Bestimmung der Lipidkonzentration in einer Probe ermöglicht. Das in der WO 2013/010970 A1 beschriebene Verfahren umfasst im Wesentlichen die Messung der Extinktion der Probe bei verschiedenen Wellenlängen, das Berechnen einer potenzfunktionellen Näherungskurve für die Extinktion der Lipide, die Subtraktion des Hämoglobin- und Bilirubin-Anteils von den Extinktionen bis eine Lipid-Kurve übrig bleibt und schließlich -zur Bestimmung des Lipid-Gehalts- die Division eines auf diese Weise theoretisch ermittelten Lipid-Extinktionswertes durch den für Lipide spezifischen Extinktionskoeffizienten.

Es wurde beobachtet, dass mit dem bekannten Verfahren in nur ca. 20 % aller Proben der Lipid-Gehalt korrekt bestimmbar ist. Bei den übrigen Proben weicht der bestimmte Lipid-Gehalt mehr als +/- 25 % vom wahren Lipid-Gehalt ab.

Der vorliegenden Erfindung liegt daher die Aufgabe zugrunde, das Verfahren gemäß WO 2013/010970 A1 so zu modifizieren, dass es eine genauere Bestimmung der Lipid-Konzentration in Körperflüssigkeitsproben ermöglicht.

Diese Aufgabe wird unter anderem dadurch gelöst, dass anstelle der Division eines bei einer Lipid-spezifischen Wellenlänge theoretisch ermittelten Lipid-Extinktionswertes durch den für Lipide spezifischen Extinktionskoeffizienten, erfindungsgemäß die Differenz von zwei Werten der Näherungskurve für die Extinktion der Lipide bei zwei unterschiedlichen Wellenlängen gebildet wird und durch den für Lipide spezifischen Extinktionskoeffizienten dividiert wird.

Dieses Vorgehen ermöglicht eine wesentlich genauere Bestimmung der Lipid-Konzentration in Körperflüssigkeitsproben. Es wird erreicht, dass der mit dem erfindungsgemäßen Verfahren bestimmte Lipid-Gehalt in keinem Fall mehr als +/- 20 % vom wahren Lipid-Gehalt abweicht. Das erfindungsgemäße Verfahren erreicht damit eine Genauigkeit der Lipid-Bestimmung, die vergleichbar ist mit der Genauigkeit einer chemischen Analyse, allerdings mit dem zusätzlichen Vorteil, dass bei dem erfindungsgemäßen Verfahren keine Reagenzien mit der Probe vermischt werden müssen.

Gegenstand der vorliegenden Erfindung ist demnach ein Verfahren zum Bestimmen der Konzentration von Lipiden in einer Körperflüssigkeitsprobe mit den Schritten:

a) Durchstrahlen der Körperflüssigkeitsprobe mit Licht bei einer Vielzahl von Wellenlängen;

b) Erfassen eines ersten Messwertes (A1) bei einer ersten Wellenlänge, bei der die nicht durch Lipide verursachte Extinktion vernachlässigbar ist;

c) Erfassen eines zweiten Messwertes (A2) bei einer zweiten Wellenlänge, bei der Bilirubin ein Extinktionsmaximum aufweist;

d) Erfassen eines dritten Messwertes (A3) bei einer dritten Wellenlänge, bei der Hämoglobin ein Extinktionsmaximum aufweist;

e) Erfassen eines vierten Messwertes (A4) bei einer vierten Wellenlänge, bei der die nicht durch Bilirubin und Hämoglobin und Lipide verursachte Extinktion vernachlässigbar ist;

f) Berechnen einer potenzfunktionellen Näherungskurve ($L_0$) der Form

$$E(\lambda) = p_0 \cdot \lambda^{q_0}$$

für die Extinktion der Lipide auf der Basis des ersten Messwerts (A1) durch Bestimmung des Faktors $p_0$ bei vorbestimmtem Exponenten $q_0$;

g) Bestimmen eines Näherungswertes der Bilirubin-Konzentration ($c_B$) auf Basis eines ersten theoretischen Extinktionswertes ($E_B$) für Bilirubin, der der Differenz zwischen dem zweiten Messwert (A2) und dem Wert der Näherungskurve ($L_0$) bei der zweiten Wellenlänge entspricht;

h) Bestimmen eines Näherungswertes der Hämoglobin-Konzentration ($c_H$) auf Basis eines zweiten theoretischen Extinktionswertes ($E_H$) für Hämoglobin, der der Differenz zwischen dem dritten Messwert (A3) und dem Wert der Näherungskurve ($L_0$) bei der dritten Wellenlänge entspricht;

i) Bestimmen eines dritten theoretischen Extinktionswertes ($E_{HBL}$) für die vierte Wellenlänge auf Basis der Summe der theoretischen Extinktionswerte für Hämoglobin ($E_H$) und Bilirubin ($E_B$) und des Wertes der Näherungskurve ($L_0$) bei der vierten Wellenlänge;

j) Ermitteln einer Abweichung des dritten theoretischen Extinktionswertes ($E_{HBL}$) von dem vierten Messwert (A4), wobei

I. sofern die in Schritt j) ermittelte Abweichung einen vorbestimmten Schwellwert nicht überschreitet- die Konzentration ($c_L$) von Lipiden bestimmt wird, indem die Differenz des Wertes der Näherungskurve ($L_0$) bei der vierten Wellenlänge und des Wertes der Näherungskurve ($L_0$) bei der ersten Wellenlänge gebildet wird und durch den für Lipide spezifischen Extinktionskoeffizienten dividiert wird, oder

II. sofern die in Schritt j) ermittelte Abweichung einen vorbestimmten Schwellwert überschreitet- eine korrigierte Näherungskurve ($L_k$) für die Extinktion der Lipide berechnet wird und die Schritte g) bis j) mit den Werten der korrigierten Näherungskurve ($L_k$) wiederholt werden bis die Abweichung den vorbestimmten Schwellwert erreicht oder unterschreitet, und die Konzentration ($c_L$) von Lipiden bestimmt wird, indem

i. sofern der erste Messwert (A1) einen vorbestimmten Extinktionsgrenzwert für die Extinktion bei der ersten Wellenlänge nicht überschreitet- die Differenz des Wertes der korrigierten Näherungskurve ($L_k$) bei der vierten Wellenlänge und des Wertes der Näherungskurve ($L_k$) bei der ersten Wellenlänge gebildet wird und durch den für Lipide spezifischen Extinktionskoeffizienten dividiert wird oder

ii. sofern der erste Messwert (A1) einen vorbestimmten Extinktionsgrenzwert für die Extinktion bei der ersten Wellenlänge überschreitet und sofern der Exponent $q_k$ der korrigierten Näherungskurve größer -1 ist- der Wert der korrigierten Näherungskurve ($L_k$) bei der ersten Wellenlänge mittels einer Ausgleichsfunktion korrigiert wird, die den ersten Messwert (A1) zum Wert der korrigierten Näherungskurve ($L_k$) bei der ersten Wellenlänge in Relation setzt, und dann die Differenz des Wertes der korrigierten Näherungskurve ($L_k$) bei der vierten Wellenlänge und des durch Anwendung der Ausgleichsfunktion korrigierten Wertes bei der ersten Wellenlänge gebildet wird und durch den für Lipide spezifischen Extinktionskoeffizienten dividiert wird.

In einer Ausführungsform des erfindungsgemäßen Verfahrens liegt die erste Wellenlänge, bei der die nicht durch Lipide verursachte Extinktion vernachlässigbar ist, zwischen 600 nm und 660 nm; vorzugsweise beträgt sie 645 nm.

In einer weiteren Ausführungsform des erfindungsgemäßen Verfahrens liegt die zweite Wellenlänge, bei der Bilirubin ein Extinktionsmaximum aufweist, zwischen 440 nm und 480 nm; vorzugsweise beträgt sie 470 nm.

In wiederum einer weiteren Ausführungsform des erfindungsgemäßen Verfahrens liegt die dritte Wellenlänge, bei der Hämoglobin ein Extinktionsmaximum aufweist, zwischen 400 nm und 440 nm; vorzugsweise beträgt sie 415 nm.

In noch einer weiteren Ausführungsform des erfindungsgemäßen Verfahrens liegt die vierte Wellenlänge, bei der die nicht durch Bilirubin und Hämoglobin und Lipide verursachte Extinktion vernachlässigbar ist, zwischen 350 nm und 370 nm; vorzugsweise beträgt sie 365 nm.

In einer bevorzugten Ausführungsform des erfindungsgemäßen Verfahrens wird der für Lipide spezifische Extinktionskoeffizient ($\varepsilon_{Lipid}$) vorab ermittelt, indem natürliches Lipid in nativen Körperflüssigkeitsproben mit bekanntem, durch chemische Analyse ermitteltem Lipid-Gehalt ($L_1$, $L_2$, ... $L_n$) mit dem erfindungsgemäßen Verfahren bestimmt wird. Dies hat gegenüber der aus dem Stand der Technik bekannten Ermittlung des Extinktionskoeffizienten ($\varepsilon_{Lipid}$), bei der Emulsionen mit künstlichem Lipid (z.B. Intralipid) verwendet werden, den Vorteil, dass die spätere Lipid-Bestimmung eine erhöhte Genauigkeit aufweist.

"Messwerte" (A1; A2; A3 etc.) im Sinne der vorliegenden Erfindung sind Extinktionsmesswerte, welche mit photometrischen Messeinrichtungen aufgenommen werden können. Bei einem Messwert kann es sich um eine dimensionslose Größe handeln, welche ein wellenlängenabhängiges Maß für die Opazität einer Körperflüssigkeitsprobe gegenüber dem Durchgang von Lichtstrahlen im sichtbaren, infraroten und/oder ultravioletten Wellenlängenbereich angibt. Es kann gleichermaßen auch möglich sein, dass Extinktionsmesswerte im Bezug auf eine Einheitsdicke einer Messzelle oder Küvette, in der Körperflüssigkeitsproben während des Durchtritts von Lichtstrahlen zur Erfassung von Intensitätsmesswerten gehalten werden, angegeben werden. In diesem Fall können die Messwerte eine Dimension von [1/cm] aufweisen. In jedem Fall sind die angegebenen Messwerte der nachfolgenden Ausführungsformen nur beispielhafter Natur und von der Messeinrichtung, der Probenbeschaffenheit und der Probenzusammensetzung abhängig. Extinktionsmesswerte werden im Folgenden jeweils mit Absorptionswerten gleichgesetzt, obwohl es dem Fachmann klar ist, dass bei dieser Betrachtung Diffraktion, Streuung und Reflexion zwar zu den Extinktionswerten beitragen, gegenüber der Absorption jedoch im betrachteten Wellenlängenbereich im Wesentlichen vernachlässigbar sind.

"Theoretische Extinktionswerte" ($E_H$, $E_B$, $E_{HBL}$ etc.) im Sinne der vorliegenden Erfindung sind nicht tatsächlich gemessene Extinktionswerte, sondern berechnete Werte.

"Lipide" im Sinne der vorliegenden Anmeldung umfassen insbesondere im menschlichen oder tierischen Organismus vorkommende Fette bzw. Triglyceride bzw. Triacylglycerine.

In einer bevorzugten Ausführungsform des erfindungsgemäßen Verfahrens wird zusätzlich die Konzentration von Hämoglobin ($c_H$) und/oder die Konzentration von Bilirubin ($C_B$) bestimmt.

Dies erfolgt im Fall I.), also wenn die in Schritt j) ermittelte Abweichung des dritten theoretischen Extinktionswertes ($E_{HBL}$) von dem vierten Messwert (A4) einen vorbestimmten Schwellwert nicht überschreitet, indem die in den Schritten g) und h) auf Basis der Näherungskurve ($L_0$) bestimmten Näherungswerte für die Hämoglobin- und die Bilirubin-Konzentrationen ($c_H$, $c_B$) als tatsächliche Hämoglobin- und die Bilirubin-Konzentrationen ausgegeben werden.

Im Fall II.), also wenn die in Schritt j) ermittelte Abweichung des dritten theoretischen Extinktionswertes ($E_{HBL}$) von dem vierten Messwert (A4) einen vorbestimmten Schwellwert überschreitet, erfolgt dies, indem die in den Schritten g) und h) auf Basis der korrigierten Näherungskurve ($L_k$) bestimmten Näherungswerte für die Hämoglobin- und die Bilirubin-Konzentrationen ($c_H$, $c_B$) als tatsächliche Hämoglobin- und die Bilirubin-Konzentrationen ausgegeben werden.

Gemäß einer vorteilhaften Ausführungsform beträgt der vorbestimmte Schwellwert für die Abweichung des dritten theoretischen Extinktionswertes ($E_{HBL}$) von dem vierten Messwert (A4) 10 mE.

Gemäß einer weiteren vorteilhaften Ausführungsform beträgt der vorbestimmte Extinktionsgrenzwert für die Extinktion bei der ersten Wellenlänge 950 mE.

Vorteilhafterweise erfolgt das Durchstrahlen der Körperflüssigkeitsprobe mit Licht bei einer Vielzahl von Wellenlängen mit Hilfe von Laser- oder Leuchtdioden oder mit Hilfe einer Lichtquelle mit verschiedenen optischen Filtern und das Erfassen der Vielzahl von Messwerten (A1; A2; A3; A4) mit Hilfe eines Photodetektors, z.B. mit einem CCD-Sensor, einem CMOS-Sensor, Photosensoren oder ähnlichen Einrichtungen, welche dazu geeignet sind, die Intensität eines Lichtstrahls wellenlängenabhängig zu erfassen.

"Körperflüssigkeitsproben" im Sinne der vorliegenden Erfindung können alle Proben biologischen Ursprungs sein, welche flüssige Konsistenz aufweisen und eine Vielzahl von biologisch aktiven Substanzen in verschiedenen Konzentrationen aufweisen. Beispielsweise können Körperflüssigkeitsproben Blutserum, Blutplasma, Blut, Urin, Lymphflüssigkeit, Gallenflüssigkeit oder ähnliche Flüssigkeiten aufweisen.

Ein weiterer Gegenstand der vorliegenden Erfindung ist ein automatisches Analysegerät mit einer Messeinrichtung, welche dazu ausgelegt ist, die Verfahrensschritte a) bis e) des erfindungsgemäßen Verfahrens auszuführen und welches ferner eine Berechnungseinrichtung, z. B. einen Prozessor, umfasst, welche dazu ausgelegt ist, die übrigen Verfahrensschritte zum Bestimmen der Konzentration ($c_L$) von Lipiden gemäß Anspruch 1 auszuführen.

In einer Ausführungsform umfasst die Messeinrichtung des automatischen Analysegeräts mindestens eine Lichtquelle und mehrere optische Filter zur Generierung von Licht unterschiedlicher Wellenlängen. In einer anderen Ausführungsform umfasst die Messeinrichtung mehrere Lichtquellen, vorzugsweise mehrere Leucht- oder Laserdioden.

In einer bevorzugten Ausführungsform umfasst die Messeinrichtung des automatischen Analysegeräts mindestens vier Lichtquellen, wobei die erste Lichtquelle Licht einer Wellenlänge im Bereich zwischen 600 nm und 660 nm emittiert, und die zweite Lichtquelle Licht einer Wellenlänge im Bereich zwischen 440 nm und 480 nm emittiert, und die dritte Lichtquelle Licht einer Wellenlänge im Bereich zwischen 400 nm und 440 nm emittiert, und die vierte Lichtquelle Licht einer Wellenlänge im Bereich zwischen 350 nm und 370 nm emittiert.

In einer besonders bevorzugten Ausführungsform umfasst die Messeinrichtung des automatischen Analysegeräts mindestens vier Lichtquellen, wobei die erste Lichtquelle Licht einer Wellenlänge von 645 nm emittiert, und die zweite Lichtquelle Licht einer Wellenlänge von 470 nm emittiert, und die dritte Lichtquelle Licht einer Wellenlänge von 415 nm emittiert, und die vierte Lichtquelle Licht einer Wellenlänge von 365 nm emittiert.

Vorteilhafterweise umfasst die Messeinrichtung außerdem mindestens einen Photodetektor, z.B. einen CCD-Sensor, einen CMOS-Sensor, Photosensoren oder ähnliche Einrichtungen, welche dazu geeignet sind, die Intensität eines Lichtstrahls wellenlängenabhängig zu erfassen.

Verschiedene Ausführungsbeispiele und Ausgestaltungen der vorliegenden Erfindung werden nun in Bezug auf die beiliegenden Zeichnungen genauer beschrieben.

FIGURENBESCHREIBUNG

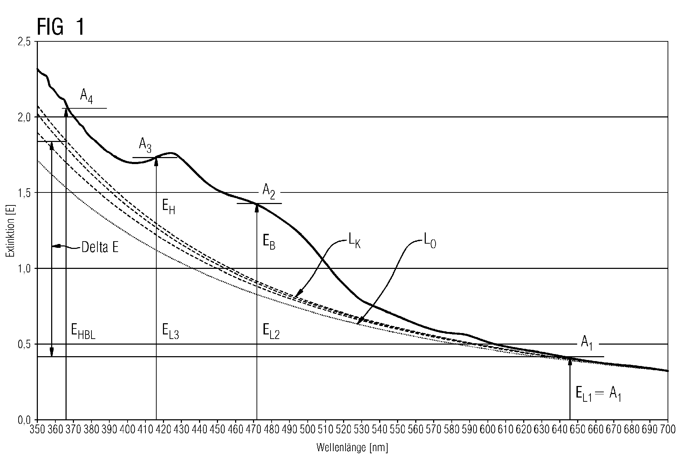

Fig. 1 zeigt eine schematische Darstellung eines Diagramms mit der Extinktionskurve einer Plasmaprobe. Die Extinktionskurve (durchgezogene Linie) mit den Messwerten A1 = $A_{645}$, A2 = $A_{470}$, A3 = $A_{415}$ und A4 = $A_{365}$ gibt einen beispielhaften schematischen Verlauf für die wellenlängenabhängige Extinktion von humanem Plasma mit Konzentrationen von Hämoglobin, Bilirubin und Lipiden wieder, deren jeweilige Extinktionen sich additiv überlagern. Die Näherungskurve $L_0$ (gepunktete Linie) für die Extinktion der Lipide wird auf Basis des ersten Messwerts A1 = $A_{645}$ und der Potenzfunktion $E(\lambda) = p_0 \cdot \lambda^{q_0}$ berechnet. Die Näherungskurve $L_k$ (gestrichelte Linie) wird berechnet und schließlich für die Berechnung der Lipid-Konzentration und der Hämoglobin- und Bilirubin-Konzentrationen herangezogen. Die Extinktionswerte der gemessenen Plasmaprobe sind vergleichsweise niedrig, wodurch sich die Näherungskurve $L_0$ durch Iteration von unten dem Extinktionsspektrum nähert bis sie die finale Näherungskurve $L_k$ erreicht.

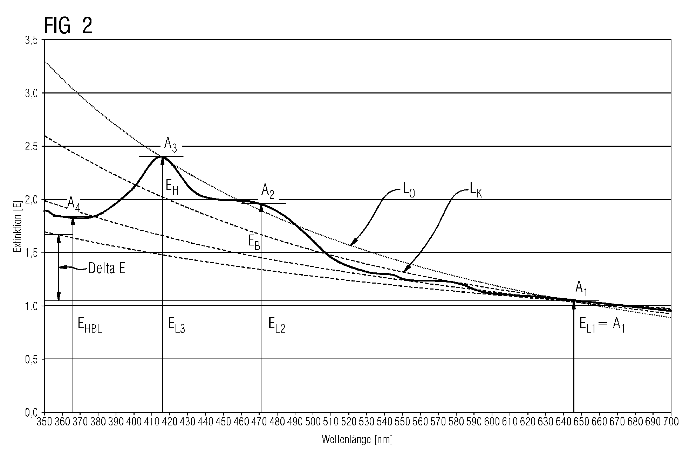

Fig. 2 zeigt wie Fig. 1 eine schematische Darstellung eines Diagramms mit der Extinktionskurve einer Plasmaprobe. Im Vergleich zu der Probe aus Fig. 1 sind hier die gemessenen Extinktionswerte vergleichsweise hoch, wodurch sich die Näherungskurve $L_0$ durch Iteration von oben dem Extinktionsspektrum nähert bis sie die finale Näherungskurve $L_k$ erreicht.

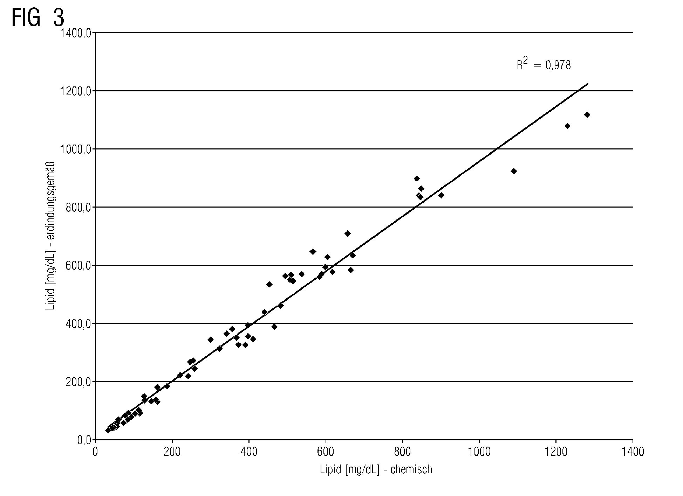

Fig. 3 zeigt ein Diagramm zur schematischen Darstellung des Vergleichs des wahren, durch chemische Analyse ermittelten Lipid-Gehalts (X-Achse) und des mit dem erfindungsgemäßen Verfahren ermittelten Lipid-Gehalts (Y-Achse) für mehrere humane Plasmaproben. Es wird deutlich, dass der erfindungsgemäß bestimmte Lipid-Gehalt in keinem Fall mehr als +/- 20 % vom wahren Lipid-Gehalt abweicht.

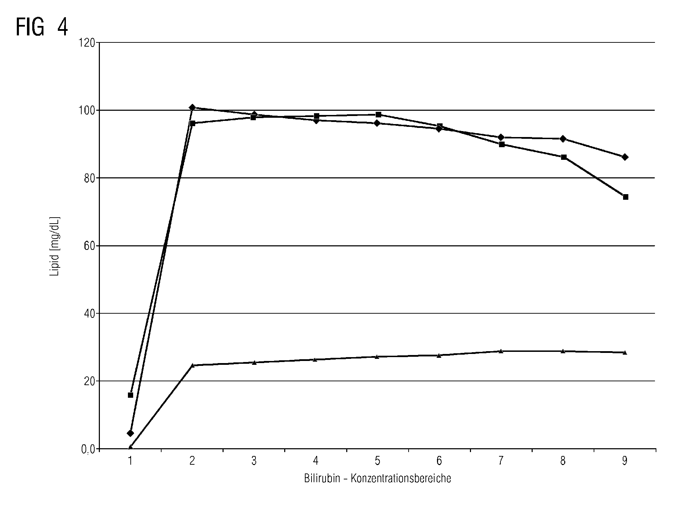

Fig. 4 zeigt ein Diagramm zur schematischen Darstellung des Vergleichs der Lipid-Bestimmung (Y-Achse) mittels chemischer Analyse (Rauten), mittels des Verfahrens gemäß dem Stand der Technik aus WO 2013/010970 A1 (Dreiecke) und mittels des erfindungsgemäßen Verfahrens (Vierecke) in Plasmaproben mit verschiedenen Bilirubin-Konzentrationen (X-Achse). Es wird deutlich, dass der erfindungsgemäß bestimmte Lipid-Gehalt im Wesentlichen mit dem chemisch bestimmten Lipid-Gehalt übereinstimmt.

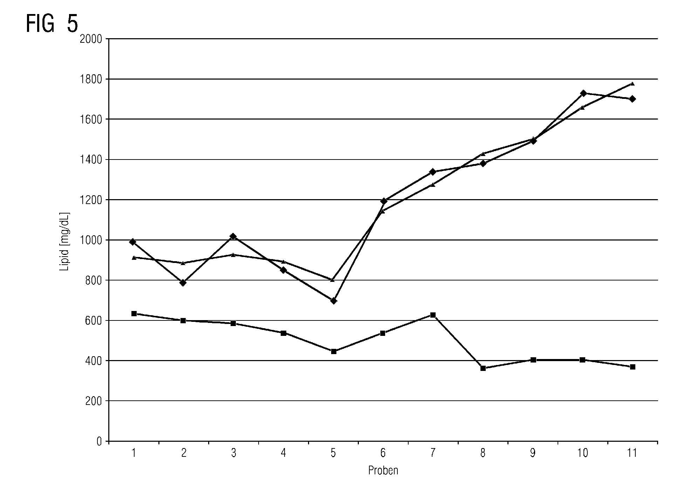

Fig. 5 zeigt ein Diagramm zur schematischen Darstellung des Vergleichs der Lipid-Bestimmung (Y-Achse) mittels chemischer Analyse (Rauten), mittels des erfindungsgemäßen Verfahrens ohne Ausgleichsfunktion (Vierecke) und mittels des erfindungsgemäßen Verfahrens mit Ausgleichsfunktion (Dreiecke) in 11 Plasmaproben mit hohen Lipid-Konzentrationen (X-Achse). Es wird deutlich, dass die erfindungsgemäße Bestimmung mit Ausgleichsfunktion eine gute Übereinstimmung mit der chemischen Analyse zeigt.

**BEISPIEL**

**a) Wellenlängen**

Das erfindungsgemäße Verfahren wurde in einem automatischen Analysegerät umfassend eine photometrische Anordnung mit vier Laserdioden durchgeführt. Humane Plasmaproben wurden mit Licht folgender Wellenlängen durchstrahlt:

645 nm    ersten Wellenlänge, bei der die nicht durch Lipide verursachte Extinktion vernachlässigbar ist;
470 nm    zweite Wellenlänge, bei der Bilirubin ein Extinktionsmaximum aufweist;
415 nm    dritte Wellenlänge, bei der Hämoglobin ein Extinktionsmaximum aufweist;
365 nm    vierte Wellenlänge, bei der die nicht durch Bilirubin und Hämoglobin und Lipide verursachte Extinktion vernachlässigbar ist.

Die Messwerte (A1 = $A_{645}$, A2 = $A_{470}$, A3 = $A_{415}$ und A4 = $A_{365}$) wurden aufgezeichnet.

**b) Lipid-spezifischer Extinktionskoeffizient**

Der für Lipid spezifische Extinktionskoeffizient $\varepsilon_{Lipid}$ wurde ermittelt, indem für 70 Plasmaproben mit bekanntem, durch chemische Analyse ermitteltem Lipid-Gehalt ($L_1$, $L_2$, ... $L_n$) mit den oben genannten Wellenlängen das Extinktionsspektrum gemessen wurde, die potenzfunktionelle Näherungskurve ($L_0$) der Form

$$E(\lambda) = p_0 \cdot \lambda^{q_0}$$

für die Extinktion der Lipide berechnet wurde mit einem vorbestimmten Exponenten $q_0$= -2,46, und der Wert der Näherungskurve ($L_0$) bei der vierten Wellenlänge ($E_{365}$) und des Wertes der Näherungskurve ($L_0$) bei der ersten Wellenlänge ($E_{645}$) gebildet wurde. Die jeweiligen Extinktionswerte wurden auf Basis der korrigierten Näherungskurve $L_k$ berechnet (siehe dazu unten, Punkt c)).

Die Differenz der Werte $E_{365}$ und $E_{645}$ dividiert durch die Lipid-Konzentration ergibt den spezifischen Extinktionskoeffizienten für Lipid. Es wurden der Mittelwert sowie der Median über alle Messungen gebildet:

$$\varepsilon_{Lipid} = \frac{1}{n}\sum_{i=1}^{n}\varepsilon = \frac{\left[\dfrac{E1_{365} - E1_{645}}{L_1}\right] + \left[\dfrac{E2_{365} - E2_{645}}{L_2}\right] + \left[\dfrac{En_{365} - En_{645}}{L_n}\right]}{n}$$

Es ergab sich der für Lipid spezifische Extinktionskoeffizient $\varepsilon_{Lipid}$ = 0,0010 dL/mg.

**c) Erstellen der potenzfunktionellen Näherungskurve für die Extinktion der Lipide und Berechnen der Konzentrationen von Lipiden und von Hämoglobin und Bilirubin**

Das Berechnen der potenzfunktionellen Näherungskurve ($L_0$) der Form

$$E(\lambda) = p_0 \cdot \lambda^{q_0}$$

für die Extinktion der Lipide erfolgt auf der Basis des ersten Messwerts (A1 = $A_{645}$) durch Bestimmung des Faktors $p_0$ bei vorbestimmtem Exponenten $q_0$.

Einzig der Messwert $A_{645}$ kann selbstverständlich noch nicht zu einer Bestimmung beider Variablen p und q dienen. Daher kann der Exponent $q_0$ anhand einer auf Referenzwerten basierenden Schätzung gebildet werden. Der Exponent $q_0$ kann demnach auf Erfahrungswerten basierend vorbestimmt werden. Bei den hier beschriebenen Bestimmungen wurde ein Exponent $q_0$ = -2,46 vorbestimmt. Da bei der ersten Wellenlänge von 645 nm die Extinktion durch andere Stoffe außer Lipiden vernachlässigt werden kann, kann bei gegebenem Exponent $q_0$ der Koeffizient $p_0$ über den Messwert A1 ($A_{645}$) bei der ersten Wellenlänge bestimmt werden. Die so ermittelte Näherungskurve mit den Parametern $p_0$ und $q_0$ kann eine erste Näherung für den Extinktionsverlauf der Extinktion von Lipiden in der Probe wiedergeben. Hierzu kann für alle Wellenlängen, in denen weitere Messwerte erfasst worden sind, der jeweilige Extinktionsanteil der Lipide berechnet werden.

Es ergibt sich dadurch eine erste Näherungskurve $L_0$, welche bereits eine gute Näherung an die tatsächliche Lipidextinktion bietet. Allerdings kann die Näherungskurve $L_0$ insbesondere in einem blauen bzw. ultravioletten Spektralbereich flacher verlaufen als der tatsächliche Extinktionsverlauf für Lipide.

Es werden dann erste Näherungswerte für die Konzentrationen von Bilirubin ($C_B$) und Hämoglobin ($c_H$) auf der Basis der Messwerte A2 und A3 bei den Wellenlängen 470 nm und 415 nm, bestimmt:

$$c_B = \frac{A3 - c_H \cdot \varepsilon_{H3} - E_{L3}}{\varepsilon_{B3}}$$

$$c_H = \frac{A2 - c_B \cdot \varepsilon_{B2} - E_{L2}}{\varepsilon_{H2}}$$

wobei $\varepsilon_{H2}$, $\varepsilon_{H3}$, $\varepsilon_{B2}$ und $\varepsilon_{B3}$ die jeweiligen Extinktionskoeffizienten von Bilirubin (B) Hämoglobin (H) bei den Wellenlängen der Messwerte A2 und A3 sind. Die Extinktionskoeffizienten können dabei vorab durch Referenzmessungen bestimmt werden, oder aus einem Speicher, in dem Referenzwerte abgelegt sind, für die Berechnungen abgerufen werden.

Zur Ermittlung der beiden Konzentrationen $c_B$ und $c_H$ kann das lineare Gleichungssystem der beiden vorgenannten Gleichungen gelöst werden, so dass sich für die Konzentration von Hämoglobin (H) die Formel

$$c_H = \frac{A3 - E_{L3} - \dfrac{(A2 - E_{L2}) \cdot \varepsilon_{B3}}{\varepsilon_{B2}}}{\varepsilon_{H3} - \dfrac{\varepsilon_{H2} \cdot \varepsilon_{B3}}{\varepsilon_{B2}}}$$

ergibt. Hierbei können die Extinktionswerte für die Lipide $E_{L2}$ und $E_{L3}$ mit der Näherungskurve $L_0$ bestimmt werden. Somit ergibt sich ein erster Näherungswert für die Konzentration $c_H$ von Hämoglobin. Dieser erste Näherungswert für die Konzentration $c_H$ kann dann zur Bestimmung des ersten Näherungswerts für die Konzentration $c_B$ von Bilirubin eingesetzt werden. Hierdurch ergeben sich bereits erste, gute Näherungswerte $c_H$, $c_B$ und $c_L$ für die Konzentrationen von Hämoglobin, Bilirubin und Lipiden, die auf Basis der Potenzfunktion und dem oben beschriebenen Gleichungssystem mit den ersten Näherungswerten für die Parameter $p_0$ und $q_0$ ermittelt wurden.

Die Näherungswerte können nun aber iterativ weiter verbessert werden, wie im Folgenden beschrieben wird. Dazu wird ein theoretischer Extinktionswert ($E_{HBL}$) für die vierte Wellenlänge auf Basis der Summe der theoretischen Extinktionswerte für Hämoglobin ($E_H$) und Bilirubin ($E_B$) und des Wertes der Näherungskurve ($L_0$) bei der vierten Wellenlänge (365 nm) bestimmt, d.h. in einem Bereich, in dem eine höhere Abweichung der tatsächlichen Lipidextinktion von der Näherungskurve zu erwarten ist:

$$E_{HBL} = c_H \cdot \varepsilon_{H1} + c_B \cdot \varepsilon_{B1} + E_{L1}$$

Die Konzentrationen $c_H$ und $c_B$ wurden oben bestimmt, der Wert $E_{L1}$ ergibt sich wieder aus der Potenzfunktion mit den Parametern $p_0$ und $q_0$.

Es wird dann ein Vergleich zwischen dem Wert $E_{HBL}$ und dem tatsächlichen Messwert A4 bei dieser Wellenlänge durchgeführt, um eine Abweichung (DeltaE)

$$\Delta E = A4 - E_{HBL}$$

zu erhalten. Wenn die Abweichung $\Delta E$ (DeltaE) größer als ein vorbestimmter Schwellwert, beispielsweise 10 mE ist, kann bestimmt werden, dass die ermittelte Näherungskurve $L_0$ für die Konzentrationen der Lipide nicht hinreichend genau genug ermittelt worden ist. In diesem Fall kann in einem weiteren Schritt ein Korrigieren der Näherungskurve $L_0$ erfolgen. Hierzu kann der berechnete Extinktionswert $E_{L1}$, welcher den Extinktionsanteil von Lipiden bei der Wellenlänge 365 nm beschreibt, um einen prozentualen Anteil der Abweichung $\Delta E$ korrigiert werden. Beispielsweise kann zu dem Extinktionswert $E_{L1}$ die Hälfte des Wertes der Abweichung $\Delta E$ addiert werden. Auf der Basis des korrigierten Extinktionswertes $E_{L1}$ kann dann eine korrigierte Näherungskurve $L_k$ mit den Parametern $p_k$ und $q_k$ bestimmt werden:

$$q_k = \frac{\ln E4 - \ln(E_{L1} + \Delta E/2)}{\ln \lambda(E4) - \ln \lambda(E1)}$$

$$p_k = \frac{E4}{\lambda(E4)^{q_k}}$$

Die Gleichungen ergeben sich dabei durch Einsetzen der Werte von E4 und dem korrigierten Wert $E1 + \Delta E/2$ in die Potenzfunktion. Dies bedeutet, dass die Näherungskurve $L_0$ derart korrigiert werden kann, dass der Messwert A1 bei der Wellenlänge von 645 nm weiterhin auf der korrigierten Näherungskurve $L_k$ liegt, das heißt, der Messwert A1 wird als Ankerpunkt für die Näherungskurve verwendet.

Im nächsten Schritt werden dann die jeweiligen Extinktionsanteile der Lipide auf der Basis der korrigierten

Näherungskurve $L_k$ berechnet. Das Verfahren wird so lange iteriert, bis bestimmt wird, dass die Abweichung den vorbestimmten Schwellwert unterschreitet. Dann werden die korrigierten Näherungswerte für die Konzentrationen von Hämoglobin, Bilirubin und Lipiden in der Körperflüssigkeitsprobe ausgegeben.

Mit dem beschriebenen Verfahren wurde der Lipid-Gehalt in allen Proben mit einer Abweichung von maximal +/- 20 % vom wahren Lipid-Gehalt bestimmt (siehe Fig. 3).

### d) Bestimmung des Lipid-Gehalts in trüben Proben

Plasmaproben, die eingefroren und wieder aufgetaut werden, weisen häufig eine Trübung auf, die eine erhöhte Extinktion zur Folge hat.

Es wurde gefunden, dass im Gegensatz zu dem aus der WO 2013/010970 A1 bekannten Verfahren mit dem erfindungsgemäßen Verfahren eine Lipidbestimmung unabhängig von der Trübung erfolgen kann.

Der Lipid-Gehalt einer Plasmaprobe (Nr. 2004657355) mit bekannter Lipid-Konzentration (667,7 mg/dL) wurde mit dem Verfahren gemäß dem Stand der Technik aus WO 2013/010970 A1 und dem erfindungsgemäßen Verfahren bestimmt. Dann wurde die Probe eingefroren, nach sechs Monaten wieder aufgetaut, und der Lipid-Gehalt wurde nochmals bestimmt. Der Gesamtextinktionsanstieg der aufgetauten Probe bei den verwendeten Wellenlängen betrug 115 %, d.h. die Probe war nach dem Auftauen trüber als vor dem Einfrieren. Die Ergebnisse sind in Tabelle 1 dargestellt. Der Tabelle ist zu entnehmen, dass mit dem herkömmlichen Verfahren nach dem Auftauen ein mehr als doppelt so hoher Lipid-Gehalt als vor dem Einfrieren gemessen wird, der jedoch immernoch mehr als 20 % vom wahren Lipid-Gehalt abweicht, während mit dem erfindungsgemäßen Verfahren nach dem Auftauen nur eine 5 %ige Abweichung gegenüber vor dem Einfrieren gemessen wird. Überdies liegt die Abweichung vom wahren Wert der Lipid-Konzentration lediglich bei -4,7 % bzw. bei 0,4 %, d.h. das erfindungsgemäße Verfahren ist grundsätzlich auch genauer.

**Tabelle 1**

|  | Vor dem Einfrieren | Nach dem Auftauen |
|---|---|---|
| **Lipid-Bestimmung gemäß Stand der Technik** |  |  |
| Konzentration | 227,8 mg/dL | 489,9 mg/dL |
| Relative Abweichung der Konzentration vom wahren Wert (667,7 mg/dL) | -66 % | -27% |
| Relative Abweichung der Konzentration nach Auftauen | --- | +115 % |
|  |  |  |
| **Lipid-Bestimmung gemäß Erfindung** |  |  |
| Konzentration | 636,6 mg/dL | 670,6 mg/dL |
| Relative Abweichung der Konzentration vom wahren Wert (667,7 mg/dL) | -4,7 % | 0,4 % |
| Relative Abweichung der Konzentration nach Auftauen | --- | +5 % |

### e) Bestimmung des Lipid-Gehalts in Gegenwart hoher Bilirubin-Konzentrationen

Plasmaproben wurden unterschiedliche Mengen Bilirubin zugesetzt, so dass 9 visuell unterscheidbare Konzentrationsstufen erhalten wurden, und der Lipid-Gehalt wurde chemisch, mit dem Verfahren gemäß dem Stand der Technik aus WO 2013/010970 A1 und mit dem erfindungsgemäßen Verfahren bestimmt. Die Ergebnisse sind in Fig. 4 dargestellt. Daraus wird deutlich, dass in Gegenwart verschiedener Bilirubin-Konzentrationen die erfindungsgemäße Lipid-Bestimmung fast genauso gut funktioniert wie die chemische Bestimmung; die größte Abweichung beträgt lediglich 7 % (Konzentrationsstufe 9).

### f) Bestimmung des Lipid-Gehalts in stark getrübten Proben

Proben mit sehr starker Trübung, beispielsweise aufgrund eines sehr hohen Lipid-Gehalts (> 500 mg/dL) weisen eine hohe Gesamtextinktion auf. Bei einigen Proben ist daher mit dem wie bisher beschriebenen erfindungsgemäßen Verfahren keine ausreichend genaue Lipid-Bestimmung möglich. Der Grund dafür ist, dass mit der Erhöhung der Lipid-Konzentration das Extinktionsspektrum der Probe zunächst gleichmäßig ansteigt, aber dann unsymmetrisch kippt, weil die Anhebung der Extinktion bei hohen Wellenlängen stärker ist als bei niedrigeren Wellenlängen. Dies hat zur Folge, dass der Extinktionsunterschied zwischen der ersten Wellenlänge (645 nm) und der vierten Wellenlänge (365 nm), die bei dem erfindungsgemäßen Verfahren verwendet werden, nicht mehr groß genug ist, um mit dem Verfahren den Lipid-Gehalt ausreichend genau bestimmen zu können. Es wurden Unterbestimmungen von bis zu -70 % beobachtet.

Dieser Effekt ist jedoch durch eine Ausgleichsfunktion korrigierbar. Die Ausgleichsfunktion korrigiert den Wert der korrigierten Näherungskurve ($L_k$) bei der ersten Wellenlänge ($E_{645}$). Die Ausgleichsfunktion ist mit einer Anzahl von Proben mit bekannter Lipidkonzentration ermittelbar. Durch Rückrechnung der Konzentration in die erwartete Extinktion kann eine Ausgleichsfunktion bestimmt werden, die es ermöglicht den wahren Lipid-Gehalt zu berechnen.

Da die Trübung stark von der Extinktion bei der Wellenlänge 645 nm abhängt, kann man den Trübungsanteil bei 645 nm berücksichtigen.

Die Berechnung wird wie folgt aufgebaut:

$$DeltaE = E_4 - \left( -0{,}7798 \cdot E_1 + 1{,}4626 \right) \cdot E_1 \Big|$$

Die Anwendung dieser Funktion auf den ersten Messwert ($A1 = A_{645}$) der Proben und der darauffolgenden Division der Differenz (Delta E) des Wertes der korrigierten Näherungskurve ($L_k$) bei der vierten Wellenlänge (365 nm) und des durch Anwendung der Ausgleichsfunktion korrigierten Wertes bei der ersten Wellenlänge (645 m) durch den für Lipide spezifischen Extinktionskoeffizienten bewirkt, dass auch in den stark getrübten Proben die Lipid-Konzentration ausreichend genau, mit einer Abweichung vom wahren Wert von weniger als +/- 20 % bestimmt werden kann. In Fig. 5 sind die Lipid-Bestimmungen für 11 Plasmaproben mit hohen Lipid-Konzentrationen vergleichend gezeigt.

## Patentansprüche

1. Verfahren zum Bestimmen der Konzentration von Lipiden in einer Körperflüssigkeitsprobe mit den Schritten:

   a) Durchstrahlen der Körperflüssigkeitsprobe mit Licht bei einer Vielzahl von Wellenlängen;
   b) Erfassen eines ersten Messwertes (A1) bei einer ersten Wellenlänge, bei der die nicht durch Lipide verursachte Extinktion vernachlässigbar ist;
   c) Erfassen eines zweiten Messwertes (A2) bei einer zweiten Wellenlänge, bei der Bilirubin ein Extinktionsmaximum aufweist;
   d) Erfassen eines dritten Messwertes (A3) bei einer dritten Wellenlänge, bei der Hämoglobin ein Extinktionsmaximum aufweist;
   e) Erfassen eines vierten Messwertes (A4) bei einer vierten Wellenlänge, bei der die nicht durch Bilirubin und Hämoglobin und Lipide verursachte Extinktion vernachlässigbar ist;
   f) Berechnen einer potenzfunktionellen Näherungskurve ($L_0$) der Form

$$E(\lambda) = p_0 \cdot \lambda^{q_0}$$

   für die Extinktion der Lipide auf der Basis des ersten Messwerts (A1) durch Bestimmung des Faktors $p_0$ bei vorbestimmtem Exponenten $q_0$;
   g) Bestimmen eines Näherungswertes der Bilirubin-Konzentration ($C_B$) auf Basis eines ersten theoretischen Extinktionswertes ($E_B$) für Bilirubin, der der Differenz zwischen dem zweiten Messwert (A2) und dem Wert der Näherungskurve ($L_0$) bei der zweiten Wellenlänge entspricht;
   h) Bestimmen eines Näherungswertes der Hämoglobin-Konzentration ($c_H$) auf Basis eines zweiten theoretischen Extinktionswertes ($E_H$) für Hämoglobin, der der Differenz zwischen dem dritten Messwert (A3) und dem Wert der Näherungskurve ($L_0$) bei der dritten Wellenlänge entspricht;
   i) Bestimmen eines dritten theoretischen Extinktionswertes ($E_{HBL}$) für die vierte Wellenlänge auf Basis der Summe der theoretischen Extinktionswerte für Hämoglobin ($E_H$) und Bilirubin ($E_B$) und des Wertes der Näherungskurve ($L_0$) bei der vierten Wellenlänge;
   j) Ermitteln einer Abweichung des dritten theoretischen Extinktionswertes ($E_{HBL}$) von dem vierten Messwert (A4) **dadurch gekennzeichnet, dass**

   I. -sofern die in Schritt j) ermittelte Abweichung einen vorbestimmten Schwellwert nicht überschreitet- die Konzentration ($c_L$) von Lipiden bestimmt wird, indem die Differenz des Wertes der Näherungskurve ($L_0$)

bei der vierten Wellenlänge und des Wertes der Näherungskurve ($L_0$) bei der ersten Wellenlänge gebildet wird und durch den für Lipide spezifischen Extinktionskoeffizienten dividiert wird, oder

II. -sofern die in Schritt j) ermittelte Abweichung einen vorbestimmten Schwellwert überschreitet- eine korrigierte Näherungskurve ($L_k$) für die Extinktion der Lipide berechnet wird und die Schritte g) bis j) mit den Werten der korrigierten Näherungskurve ($L_k$) wiederholt werden bis die Abweichung den vorbestimmten Schwellwert erreicht oder unterschreitet, und die Konzentration ($c_L$) von Lipiden bestimmt wird, indem

i. -sofern der erste Messwert (A1) einen vorbestimmten Extinktionsgrenzwert für die Extinktion bei der ersten Wellenlänge nicht überschreitet- die Differenz des Wertes der korrigierten Näherungskurve ($L_k$) bei der vierten Wellenlänge und des Wertes der Näherungskurve ($L_k$) bei der ersten Wellenlänge gebildet wird und durch den für Lipide spezifischen Extinktionskoeffizienten dividiert wird oder

ii. -sofern der erste Messwert (A1) einen vorbestimmten Extinktionsgrenzwert für die Extinktion bei der ersten Wellenlänge überschreitet und sofern der Exponent $q_k$ der korrigierten Näherungskurve größer -1 ist- der Wert der korrigierten Näherungskurve ($L_k$) bei der ersten Wellenlänge mittels einer Ausgleichsfunktion korrigiert wird, die den ersten Messwert (A1) zum Wert der korrigierten Näherungskurve ($L_k$) bei der ersten Wellenlänge in Relation setzt, und dann die Differenz des Wertes der korrigierten Näherungskurve ($L_k$) bei der vierten Wellenlänge und des durch Anwendung der Ausgleichsfunktion korrigierten Wertes bei der ersten Wellenlänge gebildet wird und durch den für Lipide spezifischen Extinktionskoeffizienten dividiert wird.

2. Verfahren nach Anspruch 1, wobei die erste Wellenlänge im Bereich zwischen 600 nm und 660 nm, und die zweite Wellenlänge im Bereich zwischen 440 nm und 480 nm, und die dritte Wellenlänge im Bereich zwischen 400 nm und 440 nm, und die vierte Wellenlänge im Bereich zwischen 350 nm und 370 nm liegt.

3. Verfahren nach Anspruch 2, wobei die erste Wellenlänge 645 nm und/oder die zweite Wellenlänge 470 nm und/oder die dritte Wellenlänge 415 nm und/oder die vierte Wellenlänge 365 nm beträgt.

4. Verfahren nach einem der vorhergehenden Ansprüche, wobei zusätzlich die Konzentration von Hämoglobin ($c_H$) und/oder die Konzentration von Bilirubin ($C_B$) bestimmt wird.

5. Verfahren nach Anspruch 4, wobei, sofern die in Schritt j) ermittelte Abweichung einen vorbestimmten Schwellwert nicht überschreitet, die Konzentrationen ($c_H$, $c_B$) von Hämoglobin und Bilirubin bestimmt werden, indem die in den Schritten g) und h) bestimmten Näherungswerte der Hämoglobin- und der Bilirubin-Konzentration als Konzentrationen ($c_H$, $c_B$) von Hämoglobin und Bilirubin ausgegeben werden.

6. Verfahren nach Anspruch 3, wobei, sofern die in Schritt j) ermittelte Abweichung einen vorbestimmten Schwellwert überschreitet, die Konzentrationen ($c_H$, $c_B$) von Hämoglobin und Bilirubin bestimmt werden, indem die in den mit den Werten der korrigierten Näherungskurve ($L_k$) wiederholten Schritten g) und h) bestimmten Näherungswerte der Hämoglobin- und der Bilirubin-Konzentration als Konzentrationen ($c_H$, $c_B$) von Hämoglobin und Bilirubin ausgegeben werden, wenn die Abweichung den vorbestimmten Schwellwert erreicht oder unterschreitet.

7. Verfahren nach einem der vorhergehenden Ansprüche, wobei der vorbestimmte Schwellwert 10 mE beträgt.

8. Verfahren nach einem der vorhergehenden Ansprüche, wobei der vorbestimmte Extinktionsgrenzwert für die Extinktion bei der ersten Wellenlänge 950 mE beträgt.

9. Verfahren nach einem der vorhergehenden Ansprüche, wobei das Durchstrahlen der Körperflüssigkeitsprobe mit Licht bei einer Vielzahl von Wellenlängen mit Hilfe von Laser- oder Leuchtdioden oder mit Hilfe einer Lichtquelle mit verschiedenen optischen Filtern erfolgt und wobei das Erfassen der Vielzahl von Messwerten (A1; A2; A3; A4) mit Hilfe eines Photodetektors erfolgt.

10. Verfahren nach einem der vorhergehenden Ansprüche, wobei die Körperflüssigkeitsprobe Serum oder Plasma ist.

11. Automatisches Analysegerät mit einer Messeinrichtung, welche dazu ausgelegt ist, die Verfahrensschritte a) bis e) gemäß Anspruch 1 auszuführen, **dadurch gekennzeichnet, dass** das Analysegerät ferner eine Berechnungseinrichtung umfasst, welche dazu ausgelegt ist, die übrigen Verfahrensschritte zum Bestimmen der Konzentration ($c_L$) von Lipiden gemäß Anspruch 1 auszuführen.

**12.** Automatisches Analysegerät gemäß Anspruch 11, wobei die Messeinrichtung mindestens eine Lichtquelle und mehrere optische Filter umfasst.

**13.** Automatisches Analysegerät gemäß Anspruch 11, wobei die Messeinrichtung mehrere Lichtquellen, vorzugsweise mehrere Leucht- oder Laserdioden umfasst.

**14.** Automatisches Analysegerät gemäß Anspruch 13, wobei die Messeinrichtung mindestens vier Lichtquellen umfasst, wobei die erste Lichtquelle Licht einer Wellenlänge im Bereich zwischen 600 nm und 660 nm emittiert, und die zweite Lichtquelle Licht einer Wellenlänge im Bereich zwischen 440 nm und 480 nm emittiert, und die dritte Lichtquelle Licht einer Wellenlänge im Bereich zwischen 400 nm und 440 nm emittiert, und die vierte Lichtquelle Licht einer Wellenlänge im Bereich zwischen 350 nm und 370 nm emittiert.

**15.** Automatisches Analysegerät gemäß Anspruch 14, wobei die erste Lichtquelle Licht einer Wellenlänge von 645 nm emittiert, und die zweite Lichtquelle Licht einer Wellenlänge von 470 nm emittiert, und die dritte Lichtquelle Licht einer Wellenlänge von 415 nm emittiert, und die vierte Lichtquelle Licht einer Wellenlänge von 365 nm emittiert.

**16.** Automatisches Analysegerät gemäß einem der Ansprüche 11-15, wobei die Messeinrichtung mindestens einen Photodetektor umfasst.

FIG 1

FIG 2

FIG 3

$R^2 = 0,978$

Lipid [mg/dL] - erdindungsgemäß

Lipid [mg/dL] - chemisch

FIG 4

Bilirubin – Konzentrationsbereiche

FIG 5

**Europäisches Patentamt**
**European Patent Office**
**Office européen des brevets**

# EUROPÄISCHER RECHERCHENBERICHT

Nummer der Anmeldung

EP 15 15 2606

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2013/010970 A1 (SIEMENS HEALTHCARE DIAGNOSTICS [DE]; SASS KARL [DE]; GREIS DIRK [DE];) 24. Januar 2013 (2013-01-24) | 11-16 | INV. G01N21/27 G01N21/31 |
| A | * Seite 1, Zeile 4 - Zeile 8 * * Seite 4, Zeile 26 - Seite 7, Zeile 36 * * Seite 12, Zeile 1 - Seite 17, Zeile 24 * * Abbildungen * | 1-10 | G01N33/49 |
| A,D | EP 1 059 522 A1 (HOFFMANN LA ROCHE [CH]) 13. Dezember 2000 (2000-12-13) * Absatz [0009] - Absatz [0019] * ----- | 1-16 | |
| A | WO 2006/040387 A1 (THERMO ELECTRON OY [FI]; JOHANSSON HENRIK [FI]) 20. April 2006 (2006-04-20) * Seite 4, Zeile 3 - Zeile 16 * * Seite 7, Zeile 16 - Seite 8, Zeile 27 * ----- | 1-16 | |
| A,D | US 2010/174491 A1 (KIM HAN SANG [KR] ET AL) 8. Juli 2010 (2010-07-08) * Absatz [0023] - Absatz [0040] * ----- | 1-16 | |

RECHERCHIERTE SACHGEBIETE (IPC)

G01N

Der vorliegende Recherchenbericht wurde für alle Patentansprüche erstellt

| Recherchenort | Abschlußdatum der Recherche | Prüfer |
|---|---|---|
| Den Haag | 13. August 2015 | Krametz, Edeltraud |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 15 2606

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-08-2015

| Im Recherchenbericht angeführtes Patentdokument | Datum der Veröffentlichung | Mitglied(er) der Patentfamilie | Datum der Veröffentlichung |
|---|---|---|---|
| WO 2013010970 A1 | 24-01-2013 | CN 103649721 A<br>EP 2549264 A1<br>EP 2710348 A1<br>JP 2014521095 A<br>US 2014192342 A1<br>WO 2013010970 A1 | 19-03-2014<br>23-01-2013<br>26-03-2014<br>25-08-2014<br>10-07-2014<br>24-01-2013 |
| EP 1059522 A1 | 13-12-2000 | EP 1059522 A1<br>EP 1185852 A1<br>JP 2003502631 A<br>US 6882425 B1<br>WO 0077494 A1 | 13-12-2000<br>13-03-2002<br>21-01-2003<br>19-04-2005<br>21-12-2000 |
| WO 2006040387 A1 | 20-04-2006 | EP 1802959 A1<br>US 2009009750 A1<br>WO 2006040387 A1 | 04-07-2007<br>08-01-2009<br>20-04-2006 |
| US 2010174491 A1 | 08-07-2010 | KR 20100082212 A<br>US 2010174491 A1 | 16-07-2010<br>08-07-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1059522 A1
- US 4263512 A
- US 20090009750 A1
- US 20100174491 A1
- WO 2013010970 A1